**Europäisches Patentamt**

**European Patent Office**

**Office européen des brevets**

(11) Publication number: **0 196 203**
**A2**

# (12) EUROPEAN PATENT APPLICATION

(21) Application number: **86302104.4**

(22) Date of filing: **21.03.86**

(51) Int. Cl.⁴: **A 61 K 45/02, A 61 K 47/00**

(30) Priority: **25.03.85 US 715851**
**03.06.85 US 740932**

(43) Date of publication of application: **01.10.86**
**Bulletin 86/40**

(84) Designated Contracting States: **AT BE CH DE FR GB IT LI LU NL SE**

(71) Applicant: **SCHERING CORPORATION, 2000 Galloping Hill Road, Kenilworth, New Jersey 07033 (US)**

(72) Inventor: **Yim, Zachary, 180 Denver Road, Paramus New Jersey 07652 (US)**
Inventor: **Chaudry, Imtiaz Ahmad, 4 Oakland Court, Denville New Jersey 07834 (US)**

(74) Representative: **Ritter, Stephen David, Mathys & Squire 10 Fleet Street, London EC4Y 1AY (GB)**

(54) **Stable gamma interferon formulation.**

(57) A stable acidic lyophilized gamma inferferon formulation and a stable frozen bulk solution of gamma interferon are disclosed.

## STABLE GAMMA-INTERFERON FORMULATIONS

The present invention relates to stable lyophilized gamma interferon formulations which upon reconstitution with water remain clear and biologically active for at least 24 hours at room temperature.

The term "gamma interferon" (GIF) refers to natural interferon or to recombinant interferon exhibiting biological properties similar to those of human immune interferon. In the disclosure of this invention, the term gamma interferon-A or GIF-A is used to denote gamma interferon containing 146 amino acids beginning with cysteine-tyrosine-cysteine and ending with glutamine. In addition, a number of truncated gamma interferon products are known, the individual truncated products being similarly designated hereinafter by an English capital letter following the term gamma interferon, e.g. GIF-D, which lacks the cysteine-tyrosine-cysteine fragment is deleted and thus has only 143 amino acids, and GIF-B, wherein GIF-A is truncated at the carboxyl terminus to a fragment containing 131 amino acids. All are contemplated for use in this invention, although a preferred form of GIF for use in this invention is GIF-A.

Gamma interferon has been reported to be an antiproliferative agent, and to potentiate the antiviral and antiproliferative effects of alpha- and beta- interferons (i.e., leukocyte and fibroblast interferons). It has been suggested that gamma interferon may also be an immunoregulatory agent.

A known method for preparing interferon dosage forms comprises lyophilizing the interferon in combination with other ingredients for reconstitution with water at the time of use. Since gamma interferon is known in the literature to be acid labile (unlike alpha-and beta-interferons, which are stable at acid pH), it has traditionally been handled at neutral or slightly alkaline pH. For example, U.K. Patent Application GB2119313A discloses lyophilized formulations containing 7 to 23 mcg/ml of GIF per ml of reconstituted solution at pH 7.5. However, such neutral or slightly alkaline solutions at higher gamma interferon concentrations (i.e. >0.2 mg/ml) are unusable as injectable formulations because of the immediate formation of a visible precipitate. Such precipitates are undesirable for several reasons, for example because of the potential for causing thrombosis in the patient, the likelihood of GIF potency loss, and the possibility of clogging the syringe needle.

The invention therefore provides a biologically stable lyophilized gamma interferon formulation comprising gamma interferon and a compatible buffer, wherein the buffer maintains the pH of a solution reconstituted with water to a concentration of from 0.001, preferably 0.05, to 10 mg gamma interferon per ml within the range of 4.0 to 6.0, preferably 4.5-5.0.

Such formulations surprisingly constitute biologically and chemically stable lyophilized powders that provide, on reconstitution with water, clear solutions.

Since GIF is preferably administered parenterally, the formulations of the present invention are primarily intended for use in injectable applications, i.e. intramuscular, intravenous and subcutaneous injections. However, such formulations will be appropriate for other methods of administering GIF wherein a GIF solution in another appropriate dosage form is necessary (e.g. nasal sprays and topical dosage forms such as ophthalmic solutions).

The acid lability of GIF is well documented. For example, J. Vilcek et al, Journal of Immunological Methods, 69 (1984), pg. 61-70, report the instability of GIF at low pH; they recovered the major portion of GIF from a column eluted at pH 3.5, but only when the fractions were collected directly into neutral buffered medium.

At low pH, solutions of GIF with or without acidic protein solubilizing agents such as human serum albumin are initially clear, in contrast to neutral or slightly alkaline solutions wherein the solution is turbid. At acid pH a precipitate does eventually form, but the presence of a solubilizing agent considerably retards precipitate formation.

The lyophilized powders of this invention remain stable at pH 4.5 after prolonged storage (e.g. one year) under refrigeration. Reconstituted solutions remain clear, i.e. free of precipitate and/or haziness, and are biologically stable for at least 24 hours at room temperature or under refrigeration. Since the powder is reconstituted just prior to use, 24 hours is more than sufficient time for the solution to remain clear for injection. Furthermore, reconstituted solutions remain clear and biologically stable after freezer storage for at least two weeks.

0196203

-4-

In the context of this invention, the term "biologically stable" means that the biological activity of the interferon formulation, as measured in the standard method by inhibition of the cytopathic effect (CPE) of a virus, is substantially retained upon storage as described above. Other less quantitative biological functions such as antiproliferative and immunoregulatory activities all show similar stability.

A significant advantage of the present invention is that at lower pH GIF is more soluble, and therefore higher concentrations than those previously disclosed for neutral or alkaline formulations of GIF are possible. At neutral pH, a >0.2 mg GIF/ml solution shows a precipitate, whereas in the formulations of the present invention GIF may be present at up to 10 mg/ml of clear reconstituted solution.

Another advantage of the present invention is that the acid pH of the formulation makes it possible to combine alpha- and/or beta-interferon, both of which are acid-stable acidic proteins, in the same formulation with gamma interferon.

Still another advantage is the ability to substantially reduce or eliminate the high concentration of salt (e.g. sodium chloride) often added to GIF solutions as a stabilizer. Lyophilization of a GIF solution low in salt produces a significantly better lyophilized powder in the preparation of dosage forms of this invention than salt-containing solutions. Also, such low-salt formulations, when reconstituted with water, form isotonic solutions suitable for intramuscular injection.

In the lyophilized formulations of the present invention, buffering agents are present such that the pH of the reconstituted solution will be 4.0-6.0, preferably 4.5 to 5.0. A preferred buffering system is citric acid

-5-

and dibasic sodium phosphate, but other organic acid buffers such as succinic acid or tartaric acid and/or inorganic acid buffers such as boric acid or one or more sodium phosphates may be used.

The formulation preferably includes a solubilizing agent, for example human serum albumin (HSA), gelatin or casein. HSA can be present in an amount up to 30 mg/ml of reconstituted solution, with 1 mg/ml being preferred.

Antioxidants, if present, may be for example sulfhydryl-specific reducing agents such as glutathione, thiodiglycolic acid, dithiothreitol and 2-mercaptoethanol, or a combination of ascorbic acid and cysteine or a pharmaceutically acceptable salt thereof. A preferred antioxidant is a combination of ascorbic acid and cysteine hydrochloride monohydrate. For GIF analogs devoid of oxidizable cysteines (e.g. GIF-D), no antioxidant is required. The antioxidant preferably is present in a range of 0.1 to 5 mg/ml reconstituted solution.

In order to form an acceptable lyophilized cake and to optimize resolubilization, one or more bulking agents such as neutral amino acids (e.g. glycine, alanine), monosaccharides (e.g. glucose), disaccharides (e.g. lactose), and polyhydric sugar alcohols (e.g. mannitol) may be included in a range of 0 to 100 mg/ml reconstituted solution, with 10-30 mg/ml being preferred. A preferred bulking agent is glycine.

Other excipients may be added to the formulation at the discretion of those skilled in the art, e.g. chelating agents such as disodium edetate in a concentration range of 0 to 0.5 mg/ml reconstituted solution.

The above-described solubilizing, antioxidant and bulking agents are merely preferred additives for the GIF formulations of the invention. The use of any or all of such agents alone or in combination is not required to obtain the advantages of the invention.

Vehicles for reconstitution of the lyophilized powder of this invention include for example water, isotonic-water and solutions comprising dextrose, glucose, propylene glycol and/or polyethylene glycol. Where the reconstituted solution is not for injection (e.g. solutions for topical or oral use), other pharmaceutically acceptable vehicles such as ethanol may be employed. A preferred vehicle for reconstitution of the lyophilized powder of this invention is water.

Another aspect of the present invention is the freeze-thaw stability of acidic bulk solutions of GIF. The term "bulk solution" refers to aqueous solutions comprising 1 to 15, preferably 1 to 10 mg GIF/ml and 0 to 5 mg/ml of an antioxidant such as cysteine, which solutions have not been prepared from lyophilized GIF. When neutral bulk solutions which may contain a maximum of 1 mg GIF/ml are frozen and thawed, copious precipitation occurs. We have surprisingly found however, that at pH 4.5 to 5.5, preferably pH 5, an 8 mg/ml bulk solution of GIF does not show precipitation upon thawing, even after prolonged storage, when stored at or below the freezing temperature of the solution (e.g. -10°C or below, -20°C being a conventional temperature at which to test stability).

Buffering agents for the bulk solution are the same as those described above for the lyophilized formulation.

0196203

-7-

For example, such a bulk solution (pH 5) may have the following composition:

| Ingredients | Concentration mg/ml |
|---|---|
| GIF | 2.0 |
| Citric Acid Anhydrous | 1.5 |
| Sodium Phosphate Dibasic Anhydrous | 1.8 |
| Cysteine HCl Monohydrate | 1.0 |
| Sodium Hydroxide 0.1N | q.s. ad pH 5 |

There are several advantages to the use of frozen acidic bulk solutions for storage of GIF. For example, the higher concentration of GIF possible at acidic pH allows for smaller volumes of solution to be stored, thus saving space; and storage of frozen material is preferable to storage of liquids since, for example, the chance of microbial growth is decreased. Also, a conventional method of storing GIF for transport in neutral 50% glycerol solution is not completely satisfactory, since the glycerol solution will freeze at the temperature used for storage during transport. Being able to freeze an acidic bulk GIF solution simplifies subsequent handling by eliminating the need for time-consuming expensive dialysis to remove the glycerol and to concentrate the solution before use. Similarly, the use of acidic bulk solutions allows for the elimination of high concentrations of salt such as sodium chloride which have been used to stabilize GIF solutions, again simplifying processing before use in formulations.

The following non-limiting example illustrates the preparation of sterile powders for preparing an injectable solution of gamma interferon:

-8-

EXAMPLE

| Ingredients | Concentration | |
| --- | --- | --- |
| | mg/vial | mg/vial |
| GIF* | 0.5 | 1.0 |
| Aminoacetic Acid | 20 | 20 |
| Citric Acid Anhydrous | 1.5 | 1.5 |
| Sodium Phosphate Dibasic Anhydrous | 1.8 | 1.8 |
| Disodium Edetate | 0.1 | 0.1 |
| Ascorbic Acid | 2 | 2 |
| Cysteine HCl Monohydrate | 0.75 | 0.5 |
| Human Serum Albumin | 1 | 1 |
| Sodium Hydroxide 0.1 N | q.s.ad pH 4.5 | q.s. ad pH 4.5 |
| Water for Injection q.s. ad | 1.0 ml | 1.0 ml |

Combine and lyophilize the ingredients using standard techniques.

*The GIF is provided as a bulk solution substantially as above described, in particular as above exemplified. The final concentration of cysteine HCl is therefore 1.0 mg/vial in each preparation.

CLAIMS:

1. A biologically stable lyophilized gamma interferon formulation comprising gamma interferon and a compatible buffer, wherein the buffer maintains the pH of a solution reconstituted with water to a concentration of from 0.001, preferably 0.05, to 10 mg gamma interferon per ml within the range of 4.0 to 6.0.

2. A formulation according to claim 1 wherein said formulation additionally contains a solubilizing agent, preferably human serum albumin in an amount of up to 30 mg per ml of reconstituted solution.

3. A formulation according to claim 1 or claim 2 wherein said formulation additionally contains an antioxidant selected from sulfhydryl-specific reducing agents and a combination of ascorbic acid and cysteine or a pharmaceutically acceptable salt thereof, preferably a combination of ascorbic acid and cysteine.

4. A formulation according to any of claims 1 to 3 wherein said formulation contains 0 to 100 mg/ml reconstituted solution of one or more bulking agents selected from neutral amino acids, monosaccharides, disaccharides, and polyhydric sugar alcohols, preferably glycine.

5. A formulation according to any of claims 1 to 4 wherein said formulation additionally contains a chelating agent, preferably disodium edetate.

6. A formulation according to any of claims 1 to 5 wherein the buffer comprises citric acid and dibasic sodium phosphate and preferably maintains a pH of 4.5 to 5.0 in the reconstituted solution.

7.      A formulation according to any of claims 1 to 6 wherein the gamma interferon is present in the range of 0.1 to 2 mg/ml reconstituted solution, preferably 1 mg/ml, and is especially gamma interferon-A.

8.      A formulation according to any of claims 1 to 7 which further contains alpha or beta interferon.

9.      A formulation comprising:

0.05 to 10 mg gamma interferon/ml of reconstituted solution,

a buffer system composed of citric acid and dibasic sodium phosphate to maintain a pH range of 4.0 to 6.0, preferably 4.5,

0 to 30 mg human serum albumin/ml of reconstituted solution, preferably 1 mg/ml,

0.1 to 5 mg ascorbic acid, preferably 2 mg, and 0.1 to 5 mg, preferably 1 mg, cysteine/ml reconstituted solution,

0 to 100 mg glycine/ml reconstituted solution, preferably 20 mg,

and 0 to 0.5 mg disodium edetate/ml reconstituted solution, preferably 0.1 mg.

10.     A reconstituted formulation according to any of claims 1 to 9 suitable for injection.

0196203

11.     A method for storing GIF comprising storing at or below its freezing temperature a solution composed of 1 to 10 mg gamma interferon and 0 to 5 mg antioxidant/ml solution and a compatible buffer which will maintain the pH of the solution in the range of pH 4.5 to 5.5, preferably about 5.0.

12.     A method according to claim 11 wherein the buffer comprises citric acid and dibasic sodium phosphate, the pH of the solution is preferably 5.0 and the antioxidant is preferably cysteine.

13.     A solution according to claim 11 or claim 12 stored at or below its freezing temperature.